Europäisches Patentamt

European Patent Office

Office européen des brevets

(19)

(11) **EP 1 047 769 B1**

(12) **FASCICULE DE BREVET EUROPEEN**

(45) Date de publication et mention
de la délivrance du brevet:
**01.09.2004 Bulletin 2004/36**

(21) Numéro de dépôt: **99900513.5**

(22) Date de dépôt: **12.01.1999**

(51) Int Cl.⁷: $C12N\ 9/02$, $A61K\ 38/44$

(86) Numéro de dépôt international:
**PCT/FR1999/000031**

(87) Numéro de publication internationale:
**WO 1999/035247 (15.07.1999 Gazette 1999/28)**

(54) **NOUVELLES UTILISATIONS THERAPEUTIQUES DES SUPEROXYDE DISMUTASES HETEROLOGUES (HSD), AINSI QUE PROCEDE DE SELECTION DESDITES HSD**

NEUE THERAPEUTISCHE VERWENDUNGEN DER HETEROLOGEN SUPEROXIDDISMUTASEN (HSD), SOWIE VERFAHREN ZUR SELEKTIERUNG DIESER HSD

NOVEL THERAPEUTIC USES OF HETEROLOGOUS SUPEROXIDE DISMUTASE (HSD), AND METHOD FOR SELECTING SAID HSD

(84) Etats contractants désignés:
**AT BE CH CY DE DK ES FI FR GB GR IE IT LI LU MC NL PT SE**

(30) Priorité: **12.01.1998 FR 9800205**

(43) Date de publication de la demande:
**02.11.2000 Bulletin 2000/44**

(73) Titulaire: **Isocell**
**92200 Neuilly sur Seine (FR)**

(72) Inventeurs:
 • **DUGAS, Bernard**
 **F-95150 Taverny (FR)**
 • **CALENDA, Alphonse**
 **F-92100 Boulogne (FR)**
 • **SAUZIERES, Jacques**
 **F-78470 Saint-Remy-les-Chevreuse (FR)**
 • **POSTAIRE, Eric**
 **F-92000 Vanves (FR)**

(74) Mandataire: **Goulard, Sophie et al**
**Cabinet ORES,**
**36,rue de St Pétersbourg**
**75008 Paris Cedex (FR)**

(56) Documents cités:
 • VAN CAMP W ET AL: "Characterization of iron superoxide dismutase cDNAs from plants obtained by genetic complementation in Escherichia coli." PROCEEDINGS OF THE NATIONAL ACADEMY OF SCIENCES OF THE UNITED STATES OF AMERICA, (1990 DEC) 87 (24) 9903-7. JOURNAL CODE: PV3. ISSN: 0027-8424., UNITED STATES, XP002081154
 • WESTENDORP ET AL: "HIV-1 TAT POTENTIATES TNF-INDUCED NF-KAPPAB ACTIVATION AND CYTOTOXICITY BY ALTERING THE CELLULAR REDOX STATE" EMBO JOURNAL, vol. 14, no. 3, 1995, pages 546-554, XP002081155 cité dans la demande
 • MACMILLAN-CROW ET AL: "NITRATION AND INACTIVATION OF MANGANESE SUPEROXIDE DISMUTASE IN CHRONIC REJECTION OF HUMAN RENAL ALLOGRAFTS" PROCEEDINGS OF THE NATIONAL ACADEMY OF SCIENCES,USA, vol. 93, 1996, pages 11853-11858, XP002081156 cité dans la demande

**Description**

La présente invention est relative à la mise en évidence des activités immuno-redox des SODs hétérologues (HSDs) et à une nouvelle utilisation thérapeutique, ainsi qu'à un procédé de sélection desdites HSDs.

Dans les conditions normales de pression d'oxygène, l'oxygène donne naissance à de l'oxygène réactif (peroxyde d'hydrogène et radicaux libres tels qu'anion superoxyde, radicaux hydroxyles ou oxyde nitrique), qui est rapidement détruit par les cellules qui disposent d'au moins trois mécanismes majeurs endogènes d'autorégulation de l'oxygène réactif produit (substances antioxydantes, chélateurs métalliques, enzymes détoxifiantes).

Par contre, si ces mécanismes sont affectés ou bien si la production d'oxygène réactif et notamment de radicaux libres est trop importante, il en résulte un stress oxydatif et l'apparition d'un état pathologique.

Les superoxydes-dismutases ou SODs, qui forment une classe de métalloprotéines contenant du fer, du cuivre, du zinc ou du manganèse, sont des enzymes capables d'induire la dismutation des anions superoxydes, pour protéger les cellules contre la toxicité de ces radicaux superoxyde ($O_2^-$).

Les anions superoxydes se forment généralement lorsque l'oxygène moléculaire acquière un électron supplémentaire, ce qui se produit lorsque l'oxygène est soumis à des radiations ionisantes. Cet anion présente une durée de vie courte et est converti en peroxyde d'hydrogène par la SOD (dismutation). Cette dismutation génère du peroxyde d'hydrogène, qui est également un oxydant cellulaire. Pour répondre à l'élévation des concentrations en peroxyde d'hydrogène, les cellules augmentent l'activité des enzymes intervenant dans l'élimination du peroxyde d'hydrogène, à savoir la catalase et la glutathion peroxidase, conformément au schéma suivant :

$$2O_2^- + 2H^+ \rightarrow H_2O_2 + O_2 \text{ superoxyde dismutase}$$

$$2H_2O_2 \rightarrow O_2 + 2H_2O \text{ catalase}$$

$$ROOH + 2GSH \rightarrow ROH + H_2O + GSSG \text{ glutathion peroxydase.}$$

En l'absence de quantités suffisantes de catalase et de glutathion peroxydase et en présence de petites quantités de fer, résultant en particulier de lésions cellulaires, on obtient une conversion du peroxyde d'hydrogène en un composé encore plus toxique, les radicaux hydroxyles (réaction de Fenton), conformément au schéma suivant :

$$O_2^- + Fe^{3+} \rightarrow Fe^{2+} + O_2$$

$$Fe^{2+} + H_2O_2 \rightarrow Fe^{3+} + OH^- + OH°$$

$$O_2^- + H_2O_2 \rightarrow O_2 + OH^- + OH°$$

Les radicaux libres dérivés de l'oxygène apparaissant impliqués dans de nombreuses affections, l'utilisation de la SOD en thérapeutique a donc été préconisée dans les pathologies induites par l'oxygène réactif telles que les états inflammatoires (arthropathies inflammatoires, par exemple) (R. NORDMANN et al., Cah. Nutr. Diet., 1991, 26, 6, 398-402) ; dans les maladies pulmonaires et plus particulièrement, la dysplasie broncho-pulmonaire ou dans d'autres conditions toxiques, liées à la présence d'oxygène en quantité importante (système nerveux central, ischémie, désordres gastro-intestinaux non vasculaires, désordres oculaires (en local dans la chambre antérieure de l'oeil) ou lutte contre les effets indésirables des traitements anti-cancéreux), la SOD a également été proposée avec plus ou moins de succès (GREENWALD R.A., Free Radical Biol. Med., 1990, 8, 201-209).

Trois types distincts de SOD, entrant dans deux familles évolutives distinctes on été décrits :

- SOD contenant du cuivre et du zinc, habituellement localisée dans le cytosol des cellules eucaryotes, dans le fluide extracellulaire des mammifères ainsi que chez certaines bactéries ;
- SOD contenant du manganèse (MnSOD) ou du fer (FeSOD), habituellement localisée chez les procaryotes ou dans les mitochondries (MnSOD) ;
- SOD contenant du fer (FeSOD), localisée dans les bactéries anaérobies et les procaryotes.

Parmi les SODs testées, ce sont celles qui présentent une demi-vie prolongée et une faible incidence des accidents de nature immunologique qui ont la préférence; on peut citer en particulier la Cu,Zn-SOD d'origine bovine (homodimère qui catalyse la dismutation du radical superoxyde), la Mn-SOD d'*E. coli*, la Fe-SOD, les SODs liposomales, les SODs conjuguées au polyéthylène glycol, les polymères ou copolymères de SOD, la Cu,Zn-SOD et la Mn-SOD humaines recombinantes ainsi que les SODs d'origine végétale.

Dans différents tests d'inhibition de l'inflammation, les SODs hétérologues s'avèrent présenter une activité anti-inflammatoire significativement plus importante que les SODs homologues.

Par exemple :

. dans un modèle de rat, dans lequel une inflammation est induite par des carraghénanes, l'activité anti-inflammatoire de différentes SODs est la suivante : Mn-SOD d'*E. coli* > Cu-SOD bovine > Cu-SOD humaine > Cu-SOD de levure > Cu-SOD de plante ; dans ce cas, la Cu-SOD de rat présente une activité pro-inflammatoire significative ;

. dans un modèle de rat, dans lequel une inflammation est induite par de l'adriamycine, l'activité anti-inflammatoire de différentes SODs est la suivante : Cu-SOD bovine > Mn-SOD d'*E. coli* ; la Cu-SOD homologue de rat est totalement inactive, alors que la Cu-SOD de levure génère une réponse pro-inflammatoire.

La Demanderesse a trouvé, de manière inattendue, qu'outre une activité dismutasique, les SODs hétérologues (HSD) possèdent une activité immuno-redox, qu'il est possible de dissocier de l'activité dismutasique.

On entend, au sens de la présente invention, par activité immuno-redox, la réactivation des défenses cellulaires internes, notamment une stimulation de la production à la fois de SOD endogène, de catalase et de glutathion peroxydase.

De telles SODs hétérologues à activité essentiellement immuno-redox, outre le fait qu'elles n'induisent pas de réaction pro-inflammatoire, induisent la production de SOD endogène et stimulent la production de catalase et de glutathion peroxydase. De telles SODs augmentent ainsi les défenses non-spécifiques, qui protègent de la dégénérescence cellulaire et organique.

La présente invention a pour objet l'utilisation d'une SOD hétérologue végétale à activité essentiellement immuno-redox, pour la préparation d'un médicament destiné au traitement des maladies dans lesquelles on observe une dégénérescence cellulaire et organique.

De telles SODs hétérologues, qui augmentent les défenses non-spécifiques, protègent les cellules de la dégénérescence ; elles constituent ainsi un médicament anti-dégénératif de choix, quelle que soit l'origine de la dégénérescence : pathologie dégénérative telle que séquelles radio-induites, maladie de Parkinson, maladie d'Alzheimer..., dégénérescence induite par un agent infectieux (SIDA, bilharziose, cirrhose post-infectieuse (hépatite C)) ou dégénérescence iatrogène (action de détoxification médicamenteuse).

Selon un mode de réalisation avantageux de ladite utilisation, ladite SOD à activité essentiellement immuno-redox est une SOD nitrée ; une telle SOD a perdu son activité dismutasique alors qu'elle conserve son activité immuno-redox.

Elle a l'avantage d'éviter tout risque d'activité pro-inflammatoire liée à l'activité dismutasique et à la production de peroxyde d'hydrogène en excès, dans le cas où l'induction de la production de catalase et de glutathion peroxydase est trop faible pour répondre à la demande.

De manière surprenante, des SODs selon l'invention (HSDs), qui ne présentent plus ou pratiquement plus d'activité dismutasique, mais qui ont conservé leur activité immuno-redox, utilisées comme médicament chez l'homme, stimulent la production de SOD endogène ainsi que la production de catalase et de glutathion peroxydase.

Selon un autre mode de réalisation avantageux de ladite utilisation, ladite SOD hétérologue végétale est notamment issue du melon.

Selon encore un autre mode de réalisation avantageux de ladite utilisation, lesdites SODs sont mises en oeuvre pour la préparation d'un médicament destiné au traitement des maladies dégénératives, sélectionnées dans le groupe constitué par les maladies neurodégénératives, la cirrhose, les infections à lentivirus, les infections parasitaires et les maladies iatrogènes (détoxification médicamenteuse).

La présente invention a également pour objet un procédé de sélection d'une SOD hétérologue végétale à activité essentiellement immuno-redox, caractérisé en ce qu'il comprend :

(a) la mesure de l'activité dismutasique d'une SOD, éventuellement modifiée,
(b) la sélection des SODs sans activité dismutasique ou ayant une activité dismutasique réduite d'au moins un facteur 10, et
(c) la mesure de l'activité immuno-redox des SODs sélectionnées en (b) sur un système cellulaire dans lequel l'expression de SOD endogène est inhibée.

Selon un mode de mise en oeuvre avantageux dudit procédé, l'étape (a) de mesure de l'activité dismutasique est réalisée par réduction du ferricytochrome c.

Selon un autre mode de mise en oeuvre avantageux dudit procédé, le système selon l'étape (c) est constitué par des cellules exprimant la protéine tat de VIH-1.

Outre les dispositions qui précèdent, l'invention comprend encore d'autres dispositions, qui ressortiront de la description qui va suivre, qui se réfère à des exemples de mise en oeuvre du procédé objet de la présente invention ainsi qu'aux dessins annexés, dans lesquels :

- la figure 1 illustre le profil électrophorétique SDS-PAGE de l'expression de SOD endogène dans des cellules U937 dans différentes conditions ;
- la figure 2 illustre l'activité de la SOD endogène des cellules U937 après activation par une SOD exogène ;
- les figures 3 (glutathion peroxydase) et 4 (catalase) illustrent les conditions dans lesquelles d'autres enzymes anti-oxydantes endogènes sont activées ;
- la figure 5 illustre l'activité dismutasique comparée de différentes SODs ;
- les figures 6 et 7 illustrent l'activité immuno-redox comparée de différentes SODs ;
- les figures 8 et 9 illustrent l'effet toxique de l'AZT, soit spontanément, soit après stimulation des CLC par des exotoxines de staphylocoque telles que la TSST-1 ;
- la figure 10 illustre l'effet toxique de plusieurs anti-rétroviraux sur des CLC ainsi que l'effet des SODs nitrées ;
- la figure 11 illustre l'effet de l'AZT sur l'apoptose spontanée ou induite par TSST-1 des cellules U1 chroniquement infectées par le VIH-1 ;
- les figures 12 et 13 illustrent l'effet de différentes SODs sur deux pathologies dégénératives : maladie d'Alzheimer et maladie de Parkinson ;
- les figures 14 et 15 illustrent des coupes histologiques de foies.
- la figure 16 illustre les effets de la présence d'anticorps anti-HSD1, dans l'activité immuno-redox de l'HSD1;
- les figures 17 et 18 illustrent les effets d'anticorps anti-HSD1 associés à une HSD1, sur la réplication du virus VIH-1 et la mort par apoptose des cellules U1 chroniquement infectées par le virus.

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée, de la présente invention.

### EXEMPLE 1 : Mise en évidence de l'activité immuno-redox des SODs.

I. Matériel et Méthodes

- Culture de cellules

La lignée cellulaire humaine leucémique promonocytaire U937 est cultivée à une concentration finale de $2.10^5$ cellules/ ml dans un milieu Iscove enrichi avec 100 U/ml de pénicilline, 100 $\mu$g/ml de streptomycine et 5 % de sérum de veau foetal, en l'absence (contrôle) ou en présence (activé) de 10 $\mu$g/ml de protéine HIV-Tat. Pour chaque expérience, 30 U/ml de SOD [recombinante humaine (HuSOD), bovine (HSD1, Sigma, St Louis, MO) ou végétale (issue du melon) (HSD2, BIO-EXTRACTION, Bron, France)] sont ajoutées au milieu et incubées à 37° C, sous une atmosphère humide comportant 5 % de $CO_2$, pendant 24 heures.

- Détermination de la SOD endogène

Les cellules U937, traitées ou non avec la protéine HIV-Tat, en présence ou en l'absence des SODs précitées, sont lysées dans un tampon détergent comprenant : 10 mM Tris-HCl pH 7,4, 0,1 % de SDS, 1 % de Nonidet P-40, 10 $\mu$g/ml de leupeptine, 100 $\mu$g/ml de PMSF et 2 $\mu$g/ml d'aprotinine, par chauffage à 85°C pendant 5 min. Le lysat est ensuite brièvement soniqué (3 x 30 sec).

Des échantillons de 20 $\mu$g sont soumis à une électrophorèse en conditions réductrices sur un gel à 12 % de SDS-polyacrylamide. Les protéines sont ensuite analysées par électroblotting sur une membrane de 0,2 $\mu$m de difluorure de vinylidène ultérieurement bloquée avec 10 mM de Tris-HCl, pH 7,4, 100 mM de NaCl (TBS) contenant 1 % de sérum albumine bovine (BSA) et 0,1 % de Tween 20.

Les empreintes sont ensuite incubées avec un antisérum de lapin anti-SOD humaine ou avec un sérum de lapin préimmun à une concentration finale de 2 $\mu$g/ml. Les empreintes sont ensuite lavées deux fois avec du TBS contenant 0,1 % de Tween 20, puis incubées avec une Ig de chèvre anti-lapin conjuguée avec de la peroxydase de raifort ; et les bandes sont révélées par chimioluminescence (luminol) et détectées par autoradiographie (film sensible à la lumière bleue).

- Détermination de l'activité dismutasique de la SOD endogène

L'activité superoxyde-dismutase totale est mesurée sur les lysats soniqués obtenus, comme décrit ci-dessus, par sa capacité à inhiber la réduction du ferrocytochrome c par les radicaux superoxydes produits par le système xanthine-oxydase/xanthine (McCord. J.M., 1969, J. Biol. Chem. 244, 6049). Une unité de superoxyde dismutase est définie comme la quantité qui inhibe 50 % de la réduction du cytochrome c, à température ambiante et à pH 7,8.

- Détermination de l'activité immuno-redox de la SOD hétérologue végétale

Après stimulation et traitement avec différentes sources et concentrations de SOD, les cellules U937 sont recueillies dans un sérum physiologique froid, tamponné au phosphate, et comptées. L'ARN total est extrait par des techniques standard (Chomczynski.P., 1987, Anal. Biochem. 162, 156), dans 1 ml de réactif TRIzol (Life Technologies ≠ 15596), selon les instructions du fabricant. Après son extraction, l'ARN est précipité avec de l'alcool isopropylique , les culots sont lavés dans de l'éthanol à 70 % et séchés à l'air. Les culots séchés sont remis en suspension dans 50 µl d'EDTA 0,5 mM, pH 8, et quantifiés par spectrophotométrie.

Pour la transcription inverse, 2 µg de l'ARN total dans 10 µl sont ensuite utilisés pour synthétiser l'ADNc, en présence de l'amorce universelle oligo-dT [Young R.A., 1983, Proc.Natl.Acad.Sci. USA, 80, 1194] dans le mélange réactionnel suivant : 1 µl de transcriptase inverse Superscript II [Life Technologies # 18089] avec 6 µl de 5x de tampon de transcription inverse (250 mM de Tris-HCl, pH 8,3, à 42°C, 50 mM de $MgCl_2$, 300 mM de KCl, 50 mM de DTT), 0,5 µl d'inhibiteur d'ARNase, 1,5 µl d'oligo-dT (1mg/ml) et 6 µl de dNTP 2,5 mM (Boehringer Mannheim).

Après une préincubation de 5 min. de l'ARN total à 65°C et un refroidissement sur de la glace, le mélange réactionnel est ajouté et la réaction a lieu pendant une heure à 42°C.

Pour l'amplification par PCR, la même quantité d'ADNc est soumise à trente cycles dans un volume réactionnel de 50 µl (25 mM de TAPS pH 9,3, 50 mM de KCl, 1 mM de $MgCl_2$, 100 µM de chaque dNTP, 20µM de chaque amorce et une unité de polymérase Goldstar).

Le programme d'amplification comporte : cinq cycles comprenant 60 sec. à 92°C, 60 sec. à 58°C et 60 sec. à 74°C, suivi de 25 cycles comprenant 30 sec. à 92°C, 60 sec. à 60°C et 60 sec. à 74°C, et pour finir la température est maintenue à 74°C pendant encore 3 minutes. Les amplifications positives PCR sont ensuite déterminées par une électrophorèse sur gel d'agarose à 1,2 % dans 0,5x de tampon TAE.

- Détermination de la transcription des gènes exprimant la catalase et la gluthation peroxydase

L'ARN cellulaire total des cellules U937 traitées ou non traitées est préparé d'après Chirgwin et al. 1979 (Chirgwin J.M., 1979, Biochemistry, 18, 5294).

Pour la transcription inverse et l'amplification par PCR, on procède comme ci-dessus.

Pour la RT-PCR, les amorces sont décrites dans la littérature, comme suit:

-    transcription de l'ARN de MnSOD : Beck. Y., 1987, Nucl. Acid. Res., 15, 9076,
-    transcription de l'ARN de Cu/ZnSOD : Sherman. L., 1995, Proc. Natl. Acad. Sci. USA, 80, 5465,
-    transcription de l'ARN de la glutathion peroxydase: Mullenbach. G.T., 1987, Nuc. Acid. Res., 15, 5484,
-    transcription de l'ARN de la catalase : Ponte.P., 1984, ibid, 12,1687/Sukenaga.Y., 1987, ibid, 15,7178/Zeviani.M., 1987, Gene, 55,205.
-    Rôle de la présence d'anticorps anti-SOD hétérologue dans l'activité immuno-redox des SODs hétérologues.

Pour montrer l'effet de synergie SOD hétérologue/anticorps anti-SOD hétérologue, les effets *in vitro* des complexes immuns HSD1/anticorps anti-HSD1 dans l'activité hétérologue des SODs ont été étudiés.

Des cellules U937, chroniquement infectées par le VIH-1 (cellules U1), ayant un taux de production de p24 faible (< 250pg/105 cellules/ml dans un surnageant de 3 jours) sont cultivées dans un milieu RPMI complémenté avec de la L-glutamine, de la pénicilline, de la streptomycine et du sérum de veau foetal à 10 % (produits Gibco). Elles sont utilisées pour la production de p24 et pour évaluer l'apoptose.

Les anticorps anti-HSD1 sont des IgG1 de souris (Valbiotech).

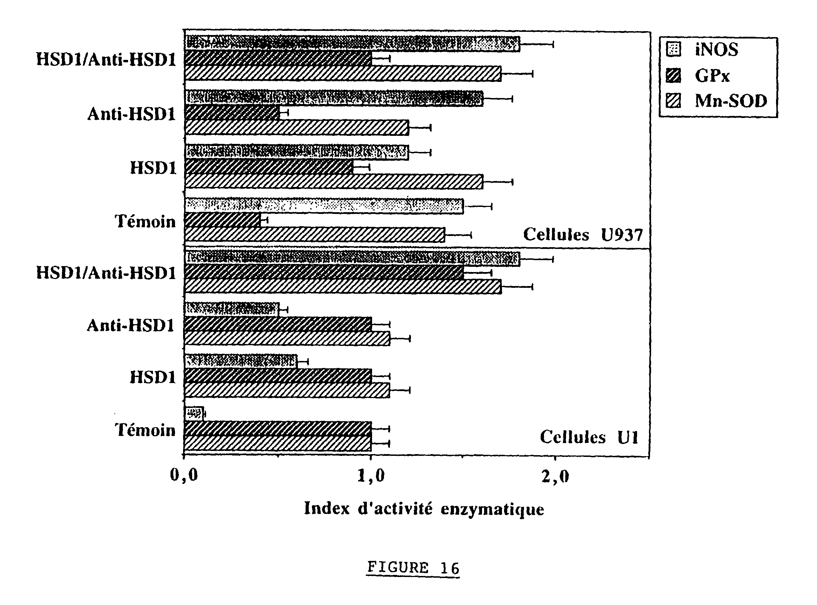

Dans les expériences effectuées (figures 16-18), l'HSD1 est utilisée à 10 µg/ml, soit 30 U/ml et les anticorps anti-HSD1 sont également utilisés à la concentration de 10 µg/ml.

II. Résultats

Détermination de l'activité immuno-redox de la SOD exogène sur des cellules stimulées U937 HIV-Tat

- Expression de la SOD endogène :

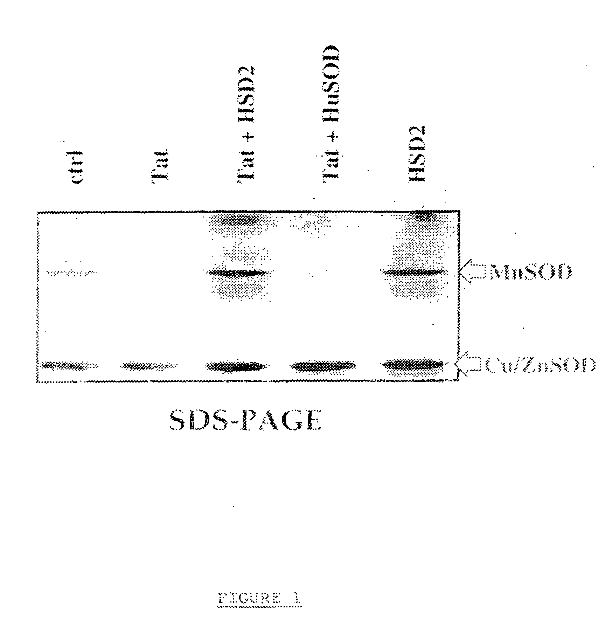

La protéine HIV-Tat est capable, à elle-seule, de réduire fortement l'expression de la protéine Mn-SOD, dans les cellules U937, sans changer celle de la Cu/Zn-SOD (Flores C.S., 1993, Proc. Natl. Acad. Sci. USA, 90, 7632). Au contraire, HSD2 (SOD végétale modifiée ou non), seule, est capable de potentialiser l'expression de base mise en évidence dans les cellules U937 non stimulées. Le résultat le plus significatif est obtenu pour les cellules activées HIV-Tat, dans lesquelles l'expression Mn-SOD reste indétectable après traitement avec la SOD humaine, alors qu'elle est rétablie après traitement avec HSD2, une forme hétérologue de SOD (figure 1).

- Activité de la SOD endogène

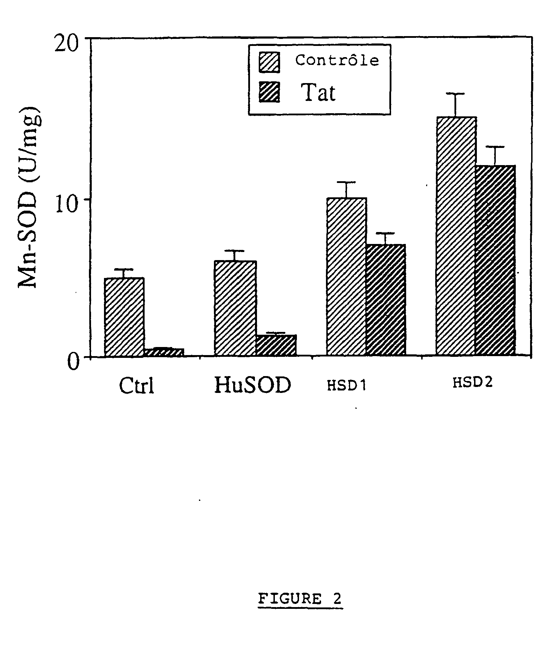

L'inhibition de l'expression de la Mn-SOD dans les cellules U937, induite par la protéine HIV-Tat, peut être levée par différentes SODs hétérologues: HSD1 (SOD bovine) ou HSD2 (SOD végétale), alors qu'elle n'est pas levée par la SOD homologue telle que la SOD humaine (HuSOD). Ceci suggère que l'inhibition de l'expression de la Mn-SOD des cellules U937, induite par la protéine HIV-Tat, peut être levée par une SOD exogène, uniquement dans un contexte hétérologue, comme l'illustre la figure 2; la Mn-SOD exprimée dans ce contexte est active.

- Activation endogène d'autres enzymes antioxydantes :

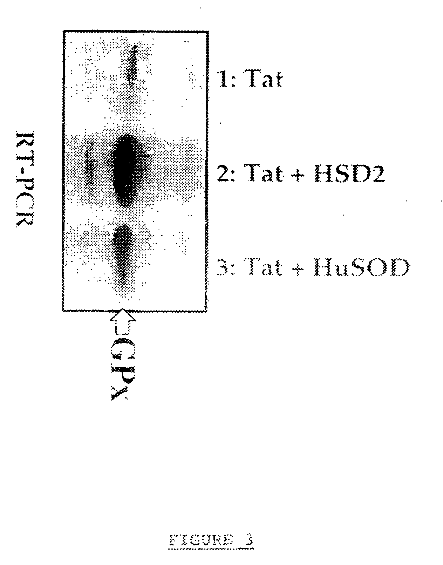

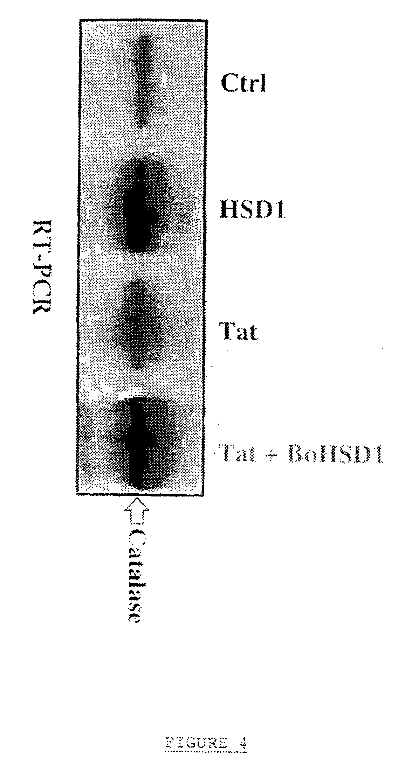

La protéine HIV-Tat diminue la quantité totale de gluthation GSH, qui est un substrat dans la réduction des peroxydes, catalysée par la gluthation peroxydase (Gpx), dans les cellules HeLa ainsi que dans les cellules Jurkat (Westerndorp M.O., 1995, EMBO J.; 14, 546).
Alors que la protéine HIV-Tat ne diminue pas la transcription de Gpx dans les cellules U937, la SOD végétale (HSD2) est capable de stimuler sa transcription, alors que HuSOD ne le permet pas, comme l'illustre la figure 3.
La même apparition d'une potentialisation par la SOD hétérologue du taux de transcription de la catalase endogène peut être observée dans des cellules non traitées autant que dans des cellules traitées par la protéine HIV-Tat, comme l'illustre la figure 4.
Ces résultats montrent que lors d'un stress oxydatif tel que celui observé dans la lignée cellulaire promonocytaire U937 pendant une simulation d'infection par l'HIV, obtenue avec la protéine HIV-Tat, les défenses antioxydantes globales et notamment la levée de l'inhibition de la Mn-SOD endogène, peuvent être restaurées par un traitement avec une SOD hétérologue, de préférence modifiée, de manière à ne conserver que son activité immuno-redox, alors qu'elles sont peu modifiées par un traitement avec la SOD homologue.

- effets des complexes immuns HSD1/anticorps anti-HSD1 dans l'activité immuno-redox des SODs hétérologues.

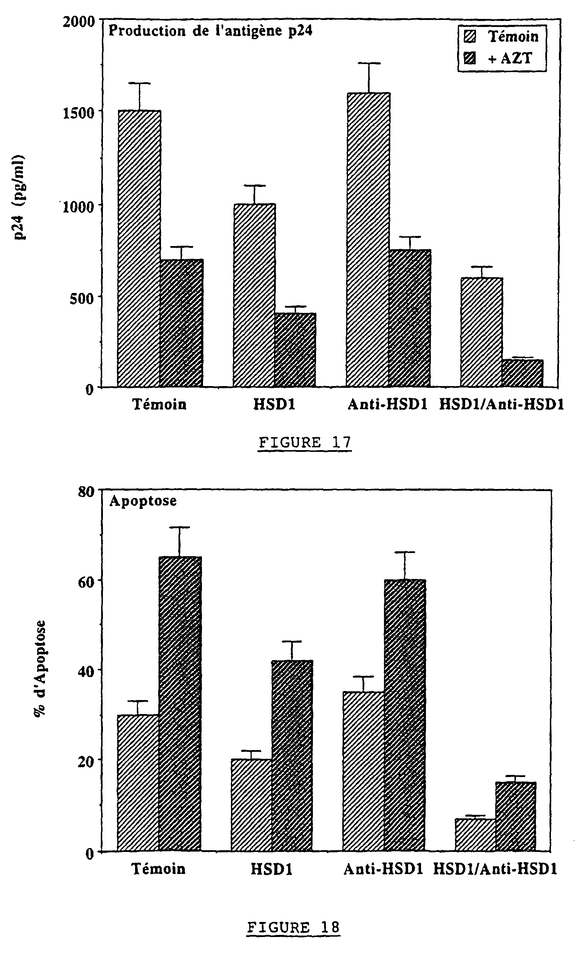

Lorsque des cellules macrophagiques U937 non infectées et des cellules U1 infectées chroniquement par le VIH-1 sont stimulées par le complexe immun HSD1/anti-HSD1, on obtient les résultats illustrés à la figure 16 : on observe que si les HSD1 stimulent l'expression de la glutathion peroxydase (GPx), de la Mn-SOD et de la NO synthase inductible (iNOs), cette stimulation se trouve être significativement augmentée dans le cas où l'HSD1 est complexée avec un anticorps anti-HSD1. De plus, ce même complexe immun réduit de manière significative la réplication du virus VIH-1 (production de p24) par les cellules U1 stimulées par 10 ng/ ml de TNF et renforce les effets d'une dose sous-optimale d'AZT (1 µg/ml) (figure 17) ; de la même manière, ce complexe réduit la mort par apoptose des cellules U1 stimulées par 10 ng/ml de TNF, en présence ou non d'AZT (figure 18) (voir également exemple 3).
Ces résultats montrent que les complexes immuns HSD1/anti-HSD1 renforcent de manière significative les effets pharmacologiques des HSD1. Ainsi on peut obtenir un effet pharmacologique optimal avec les SODs hétérologues puisque la stimulation du système immunitaire, après injection ou ingestion, induit une activité immunologique de soutien (production d'anticorps anti-HSD1).

**EXEMPLE 2 : Mise en évidence de la perte de l'activité dismutasique d'une SOD modifiée (SOD nitrée).**

I. Matériel et méthodes

- mesure de l'activité glutathion peroxydase : voir exemple 1.
- mesure de la Mn-SOD : voir exemple 1.
- cellules cibles : cellules lymphoïdes circulantes ou CLC, cultivées dans les mêmes conditions que celles exposées à l'exemple 1 pour les cellules U937.
- préparation de la SOD nitrée : elle est préparé conformément à la méthode décrite dans l'Article au nom de L.A. MacMillan-Crow, paru dans Proc. Natl. Acad. Sci. USA, 1996, 93,11853-11858.

II. Résultats

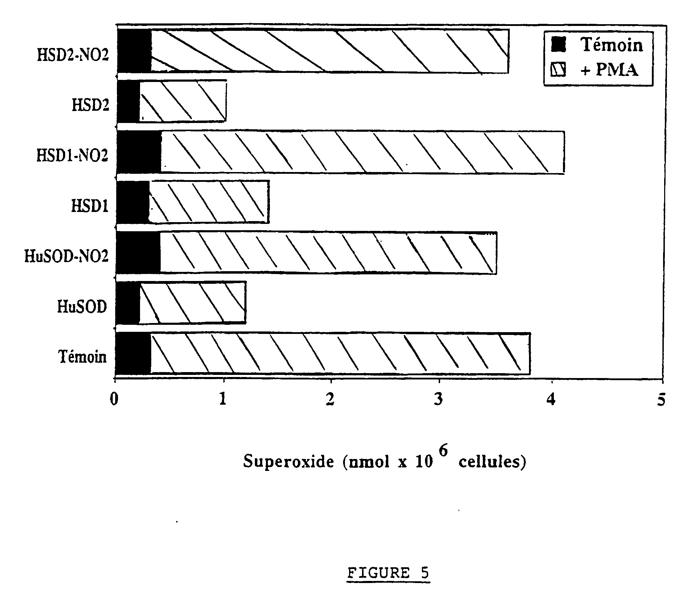

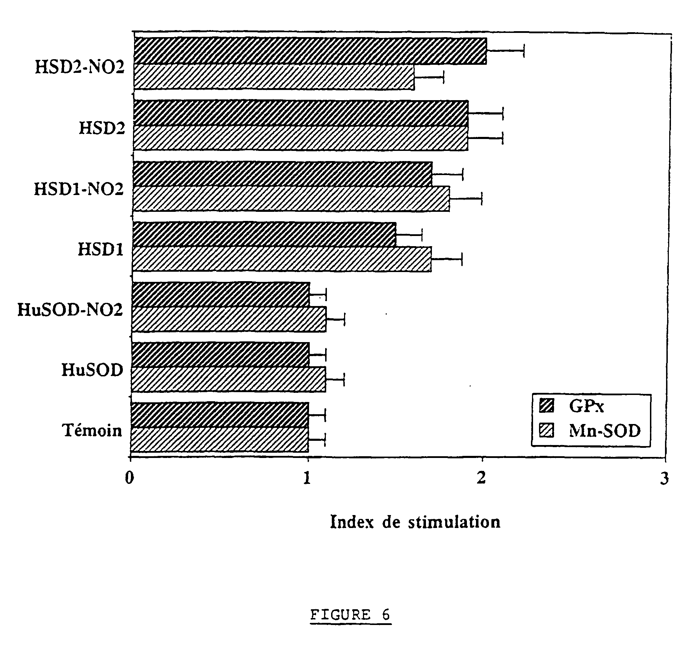

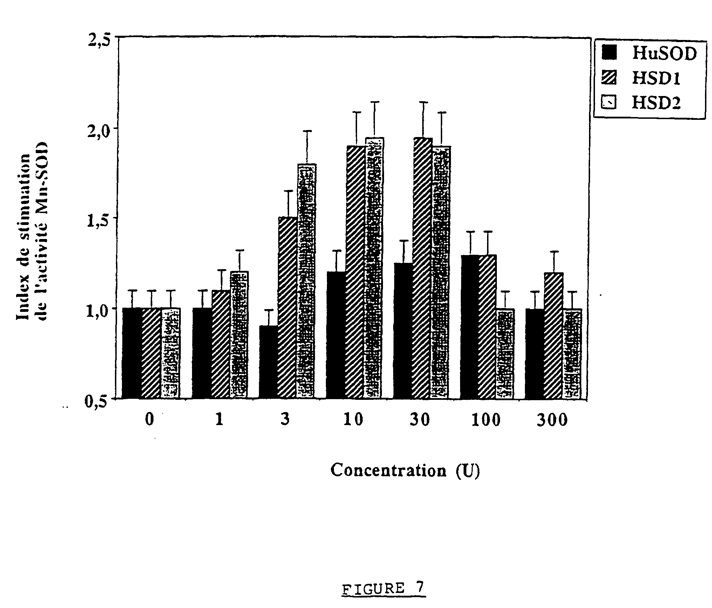

La figure 5 montre que les SODs nitrées ont perdu l'activité dismutasique ; en effet, l'administration de SOD à activité essentiellement immuno-redox n'a pas d'action sur la production d'anions superoxydes induite par un ester de phorbol (PMA) dans des cellules lymphoïdes circulantes ou CLC, alors que l'on observe une stimulation de la glutathion peroxydase et de la Mn-SOD endogène (figure 6 et figure 7), dans ces mêmes cellules cibles.

**EXEMPLE 3: Effets des SODs sur une dégénérescence cellulaire d'origine iatrogène; cas des antiviraux.**

I. Matériel et méthodes

- Réactifs

\* Des cellules U937, chroniquement infectées par le VIH-1 (cellules U1), ayant un taux de production de p24 faible (< 250 pg/$10^5$ cellules/ml dans un surnageant de 3 jours) sont cultivées dans un milieu RPMI complémenté avec de la L-glutamine, de la pénicilline, de la streptomycine et du sérum de veau foetal à 10 % (produits Gibco). Elles sont utilisées pour la production de p24 et pour évaluer l'apoptose. Le milieu de culture, les produits chimiques et le sérum de veau foetal sont testés pour leur absence d'effet direct sur ces cellules U1 (expression de TNF-$\alpha$ et de p24, comme marqueurs d'activation). Les cultures sont également complémentées avec : TSST-1 (exotoxine de staphylocoque) (Sigma, Paris, France), de la SOD humaine Cu/Zn (HuSOD), de la SOD bovine (HSD1) (Sigma, St Louis, MO) ou de la SOD de melon Cu/Zn (HSD2), fournie par la Société BIO-EXTRACTION (Bron, France).

Ces cellules U1 sont stimulées avec du TSST-1 (10 $\mu$g/ml), de la ZINOVUDINE (AZT) (Welcome) (1 à 10 $\mu$g/ml), du SAQUINAVIR (Roche) ou du RITONAVIR (Abbott) (10 $\mu$g/ml), pendant 48 heures.

Dans les expériences de contrôle, les produits anti-rétroviraux sont testés seuls.

Quelques cultures sont également complémentées avec HuSOD, HSD1 ou HSD2, à une concentration finale de 30 U / ml.

Une analyse préliminaire utilisant différentes concentrations de ces réactifs, a permis de définir ces doses optimales.

Après 1 à 3 jours d'incubation, les surnageants de cellules sont récupérés et les taux de p24, de nitrites et de TNF-$\alpha$ sont déterminés.

Les cellules viables sont comptées par exclusion au bleu trypan.
\* Les protéines HIV-p24 (Institut Pasteur)et TNF-$\alpha$ (Genzyme) sont mesurées par ELISA, selon les recommandation du fabricant.
\* NO et $NO_2^-$ sont mesurés par la réaction de Greiss.

II. Tests

- Cultures de CLC pour l'évaluation de TSST-1 et de HSD

Isolement et culture de cellules mononucléées humaines du sang périphérique (CLC), qui sont utilisées pour évaluer la production de nitrites, de TNF-$\alpha$ et l'apoptose.
Les CLC sont isolées à partir de 10 sujets, par centrifugation à 900 rpm pendant 10 min., pour éliminer la majorité des plaquettes contaminantes. Les échantillons sanguins sont ensuite dilués dans un milieu RPMI 1640 (Bioproduct) et les CLC sont récupérées après une centrifugation de 20 min à 2 000 rpm sur un gradient Ficoll-Hypaque (Pharmacia).

Ces donneurs ne présentent aucune infection (hépatite ou SIDA), ni cirrhose ; certains des donneurs présentent des rhinites ou un état asthmatique mais ont été testés en dehors des périodes de pollinisation et en l'absence de tout traitement.

Les CLC sont cultivées à une concentration cellulaire finale de $2.10^6$ cellules/ml dans des plaques de culture en plastique comprenant 12 ou 24 puits (Nunc) et dans un milieu Iscove complémenté avec 100 U/ml de pénicilline, 100 µg/ml de streptomycine, 1,8 µg/ml d'éthanolamine, 40 µg/ml de transferrine, 5 µg/ml d'insuline, 2 µg/ml d'acide linoléique, 2 µg/ml d'acide oléique, 2 µg/ml d'acide palmitique, 0,25 % de sérum albumine bovine et 5 % de sérum de veau foetal.

Ce milieu de culture ne contient pas d'endotoxine (estimation avec un test au limulus).

Les cellules sont incubées à 37°C en atmosphère humide (100 %) comprenant 95 % d'air et 5 % de $CO_2$, pendant 2-6 jours en présence ou en l'absence de TSST-1 (10 µM) et de HuSOD, HSD1 ou HSD2 (30 U/ml).

Des expériences similaires sont effectuées pour évaluer l'effet toxicologique potentiel de l'AZT, du SAQUINAVIR ou du RITONAVIR (10 µg/ml) et l'effet protecteur des SODs (HuSOD, HSD1 et HSD2 à 30 U/ml).

- Dosage des nitrites ($NO_2^-$).

Pour évaluer la quantité d'oxyde nitrique produite ($NO°$), on dose, dans les surnageants de culture, les nitrites ($NO_2^-$), un produit final stable obtenu à partir de l'oxyde nitrique, par la réaction de Griess. 100 µl de surnageant sont ajoutés dans des plaques de microtitration comportant 96 puits, ainsi que 100 µl d'une solution réactive composée de sulfinamide à 1 % dans de l'acide acétique à 30 % et de dihydrochlorure de N-1-naphtyléthylènediamine à 0,1 % dans de l'acide acétique à 60 %.

La courbe standard est effectuée avec du $NaNO_2$ dilué dans du milieu Iscove. Les densités optiques (OD) sont mesurées à 540 nm (lecteur Dynatech laboratories Inc., Alexandria, VA).

- Mesure de l'apoptose

Les cellules U1 ou les cellules CLC en apoptose précoce sont estimées par une détection cellulaire par fluorescence (FACS ou *fluorescence-activated cell sorting*) de la phosphatidylsérine en présence d'annexine V marquée au FITC, avec un marquage simultané ou non au iodure de propidium. La fragmentation d'ADN induite par l'apoptose est aussi estimée, avec le kit de détection de la fragmentation de l'ADN ApoALERT (Clontech, Palo Alto, CA).

III. Résultats

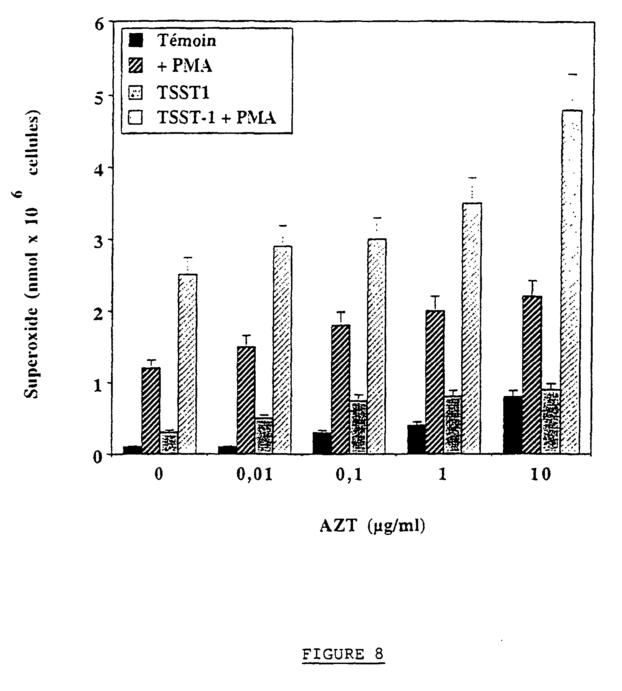

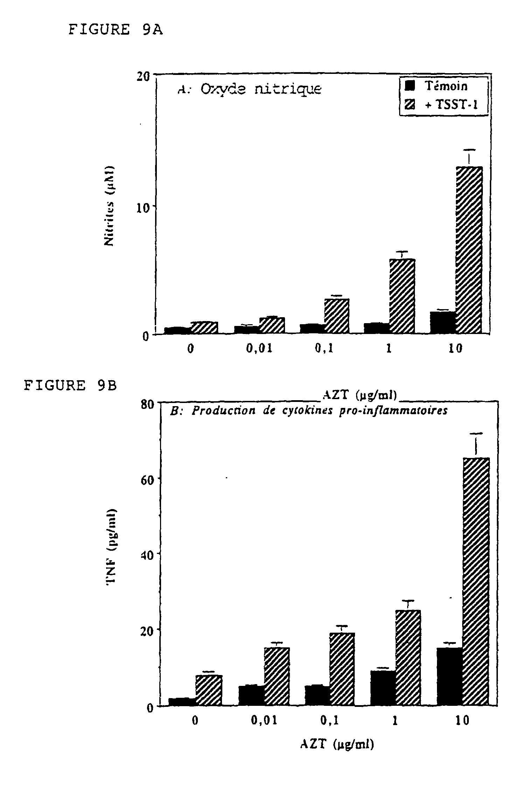

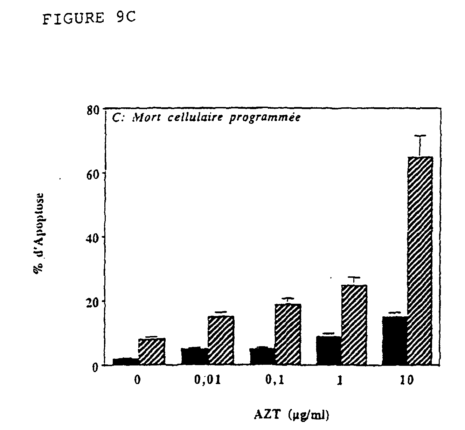

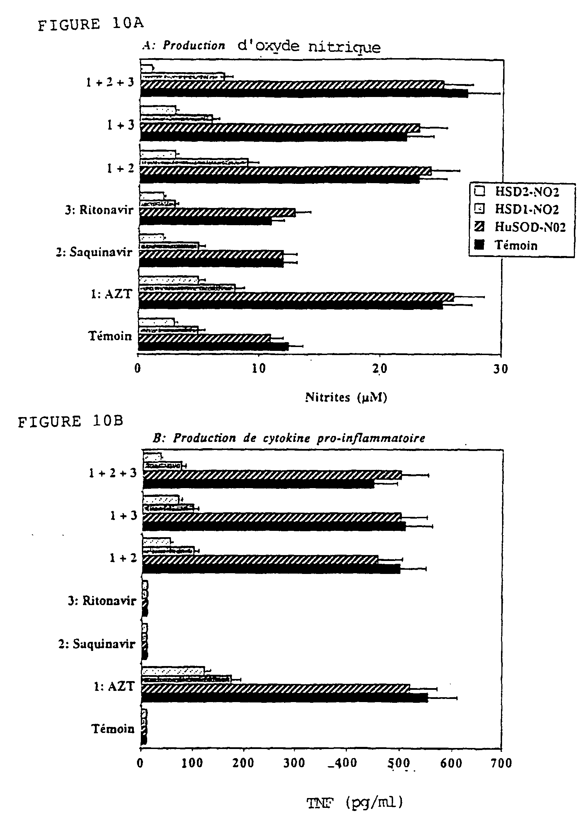

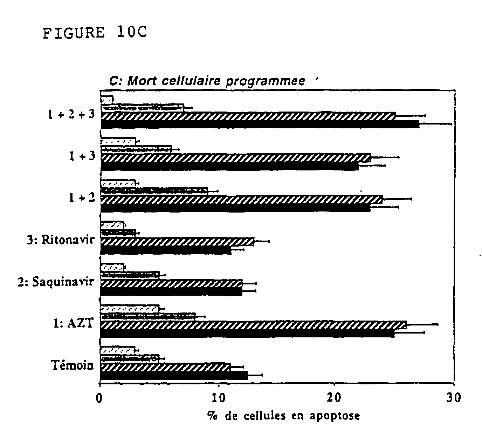

- les figures 8 et 9 illustrent l'effet toxique de l'AZT (figure 8 et figure 9), soit spontanément, soit après stimulation des CLC par des exotoxines de staphylocoque telle que la TSST-1; la figure 10 illustre l'effet toxique de plusieurs antiviraux sur des CLC ainsi que l'effet des SODs nitrées ; on observe, sur cette figure 10 l'effet des SODs nitrées sur la production d'oxyde nitrique (mesure des nitrites (µM)), la production de cytokine pro-inflammatoire (mesure de TNF (ng/ml) et l'évaluation de la mort cellulaire (% de cellules en apoptose) sur des cellules non-infectées (CLC) sous traitement (monothérapie, bithérapie et trithérapie).
- Le Tableau I ci-après illustre l'effet de l'AZT sur la réplication virale du VIH-1 et sur le statut pro-inflammatoire des cellules chroniquement infectées (U1).

Tableau I

| AZT | Superoxyde (+PMA) | | Oxyde nitrique | | TNF | | Antigène p24 | |
|---|---|---|---|---|---|---|---|---|
| (µg/ml) | Témoin | +TSST- | Témoin | +TSST-1 | Témoin | +TSST-1 | Témoin | +TSST-1 |
| 0 | 1,1* | 2,5* | 0,5 | 5,5 | <10 | 275 | 900 | 2512 |
| 0,01 | 1,5 | 3,3 | 0,8 | 6,1 | <10 | 280 | 750 | 2367 |
| 0,1 | 2,3 | 3,5 | 0,9 | 7,0 | <10 | 325 | 620 | 1235 |

* Les témoins sans PMA sont 0,3 sans TSST-1 et 0,5 avec TSST-1
oxyde nitrique : les résultats sont exprimés en µM de nitrites
TNF : les résultats sont exprimés en pg/ml
antigène p24 : les résultats sont exprimés en pg/ml.

Tableau I (suite)

| AZT | Superoxyde (+PMA) | | Oxyde nitrique | | TNF | | Antigène p24 | |
|---|---|---|---|---|---|---|---|---|
| (µg/ml) | Témoin | +TSST- | Témoin | +TSST-1 | Témoin | +TSST-1 | Témoin | +TSST-1 |
| 1 | 2,5 | 4,2 | 0,9 | 12,2 | 55 | 401 | 312 | 809 |
| 10 | 2,9 | 5,1 | 0,9 | 15,3 | 95 | 805 | 100 | 312 |

Les résultats sont calculés à partir d'une série d'expériences représentatives parmi 5 différentes et sont la moyenne de quadruplicats, l'écart à la moyenne n'excèdent pas 10 %.

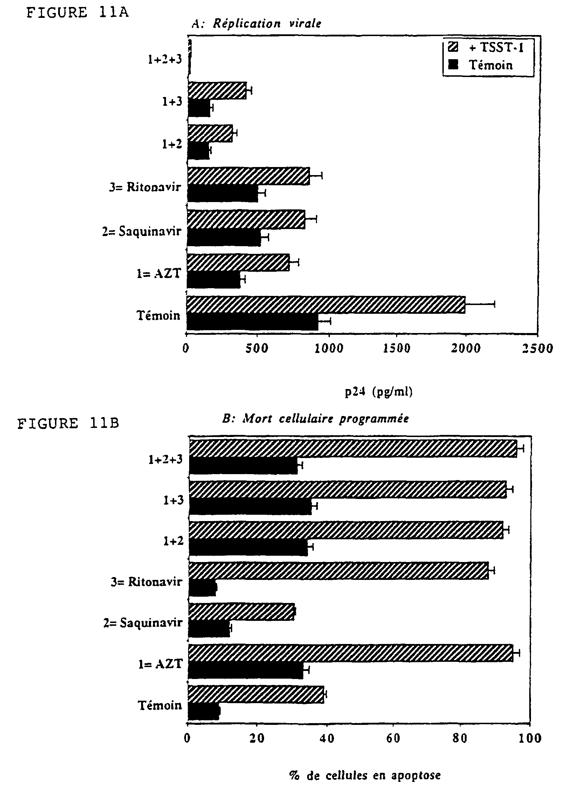

- Le Tableau II et la figure 11 illustrent l'effet de l'AZT sur l'apoptose spontanée ou induite par TSST-1 des cellules U1 chroniquement infectées par le VIH-1.

Tableau II

| AZT (µg/ml) | % de cellule en apoptose | |
|---|---|---|
| | Témoin | +TSST-1 |
| 0 | 7 ± 2 | 24 ± 3 |
| 0,01 | 9 ± 1 | 27 ± 1 |
| 0,1 | 17 ± 4 | 42 ± 3 |
| 1 | 25 ± 5 | 61 ± 2 |
| 10 | 29 ± 4 | 90 ± 4 |

Les résultats exprimés en % de cellules en apoptose (Kit Apoptag) représentent la moyenne ± SEM de quatre expériences différentes.

\* effet des SODs non-nitrées et nitrées sur le statut inflammatoire des cellules infectées par le HIV-1, traitées ou non par un antiviral.

Le Tableau III illustre l'effet de la HuSOD, de la HSD1 et de la HSD2 sur la réplication virale et la mort cellulaire programmée des cellules UI chroniquement infectées par le VIH-1 (en présence ou en l'absence d'antiviraux).

Tableau III

| Stimulation | p24 (pg/ml) | % d'apoptose |
|---|---|---|
| **A : sans agent anti-rétroviral** | | |
| Témoin | 950 ± 25 | 15 ± 2 |
| HuSOD | 700 ± 12 | 10 ± 1 |
| HSD1 | 550 ± 32 | 8 ± 1 |
| HSD2 | 480 ± 23 | 6 ± 2 |
| TSST-1 | 1756 ± 75 | 89 ± 5 |
| TSST-1 + HuSOD | 1290 ± 45 | 75 ± 2 |
| TSST-1 + HSD1 | 900 ± 56 | 30 ± 4 |
| TSST-1 + HSD2 | 923 ± 78 | 34 ± 7 |
| **B : en présence d'AZT** | | |
| Témoin | 233 ± 12 | 45 ± 2 |
| HuSOD | 200 ± 27 | 35 ± 1 |
| HSD1 | 125 ± 32 | 18 ± 1 |
| HSD2 | 130 ± 26 | 11 ± 2 |
| TSST-1 | 1056 ± 45 | 98 ± 1 |
| TSST-1 + HuSOD | 890 ± 21 | 70 ± 2 |
| TSST-1 + HSD1 | 300 ± 76 | 17 ± 4 |
| TSST-1 + HSD2 | 223 ± 78 | 15 ± 7 |

Tableau III   (suite)

| Stimulation | p24 (pg/ml) | % d'apoptose |
|---|---|---|
| **C : en présence de Saquinavir** | | |
| Témoin | 763 ± 45 | 15 ± 2 |
| HuSOD | 301 ± 12 | 13 ± 1 |
| HSD1 | 223 ± 33 | 9 ± 1 |
| HSD2 | 170 ± 21 | 10 ± 2 |
| TSST-1 | 925 ± 54 | 75 ± 5 |
| TSST-1 + HuSOD | 700 ± 29 | 65 ± 2 |
| TSST-1 + HSD1 | 313 ± 24 | 24 ± 5 |
| TSST-1 + HSD2 | 208 ± 21 | 19 ± 7 |
| **D : en présence de Ritonavir** | | |
| Témoin | 843 ± 56 | 15 ± 2 |
| HuSOD | 658 ± 12 | 10 ± 1 |
| HSD1 | 221 ± 11 | 4 ± 1 |
| HSD2 | 172 ± 19 | 6 ± 2 |
| TSST-1 | 1025 ± 62 | 70 ± 3 |
| TSST-1 + HuSOD | 810 ± 13 | 59 ± 4 |
| TSST-1 + HSD1 | 203 ± 12 | 34 ± 2 |
| TSST-1 + HSD2 | 228 ± 11 | 22 ± 3 |
| **E : AZT + Saquinavir + Ritonavir** | | |
| Témoin | 203 ± 13 | 45 ±2 |
| HuSOD | 150 ± 10 | 30 ± 3 |
| HSD1 | 70 ± 12 | 10 ± 1 |
| HSD2 | 82 ± 14 | 10 ± 2 |
| TSST-1 | 275 ± 24 | 85 ± 3 |
| TSST-1 + HuSOD | 200 ± 21 | 62 ± 3 |
| TSST-1 + HSD1 | 88 ± 10 | 10 ± 2 |
| TSST-1 + HSD2 | 87 ± 11 | 5 ± 1 |

Le Tableau IV illustre l'effet de la HuSOD, de la HSD1 et de la HSD2 sur le statut inflammatoire des cellules U1 chroniquement infectées par le VIH-1

## Tableau IV

| Traitement : | Nitrites (µM) | | TNF (pg/ml) | |
|---|---|---|---|---|
| | Témoin | +TSST-1 | Témoin | +TSST-1 |
| Témoin | 1 ±0,5 | 12 ± 3 | < 10 | 1256 ±98 |
| 1 : AZT | 5 ± 0,2 | 33 ± 4 | 175 ±23 | 2934 ± 76 |
| 2 : Saquinavir | 1 ± 0,2 | 11 ± 2 | < 10 | 1050 ± 72 |
| 3 : Ritonavir | 1 ± 0,4 | 10 ± 2 | < 10 | 1001 ± 45 |
| 1 + 2 | 1 ± 0,2 | 13 ± 4 | < 10 | 1154 ± 87 |
| 1 + 3 | 1 ± 0,1 | 14 ± 2 | < 10 | 1212 ± 94 |
| 1 + 2 + 3 | 7 ± 0,4 | 29 ± 3 | 178 ± 21 | 2765 ± 34 |
| 4 : HuSOD | 1 ± 0,2 | 9 ± 2 | < 10 | 1145 ± 12 |
| 1 + 4 | 4 ± 0,3 | 20 ± 4 | 154 ± 22 | 975 ± 44 |
| 1 + 2+ 3 + 4 | 6 ± 0,2 | 24 ± 5 | 109 ± 17 | 999 ± 65 |
| 5 : HSD1 | 1 ± 0,2 | 3 ± 1 | < 10 | 221 ± 21 |
| 1 + 5 | 2 ± 0,3 | 9 ± 1 | 55 ± 10 | 175 ± 22 |
| 1 + 2 + 3 + 5 | 1 ± 0,4 | 5 ± 1 | 23 ± 2 | 108 ± 11 |
| 6 : HSD2 | 1 ± 0,1 | 4 ±2 | < 10 | 209 ± 45 |
| 1 + 6 | 1 ± 0,2 | 11 ± 4 | 33 ± 2 | 200 ± 14 |
| 1 + 2 + 3 + 6 | 1 ± 0,3 | 6 ± 1 | 25 ± 4 | 109 ± 19 |

Les Tableaux III et IV montrent que sous l'action de la TSST-1 seule ou en présence d'agents anti-rétroviraux, on note une forte augmentation de la réplication virale et de l'apoptose, alors que les HSDs réduisent significativement ces effets ; par contre, la HuSOD n'a pas ou peu d'effets.

Ces résultats démontrent que les HSDs renforcent les effets des anti-rétroviraux tout en inhibant leurs effets toxiques.

**EXEMPLE 4 : Effets des SODs sur une pathologie dégénérative ; cas de la maladie d'Alzheimer et de la maladie de Parkinson.**

I. Matériel et méthodes

- culture pour l'évaluation des effets des protéines β-amyloïdes et MPTP

* Isolement des macrophages humains.

Les CLC sont isolées à partir de 23 sujets sains ; dans les mêmes conditions que celles exposées ci-dessus. Les populations de cellules adhérentes sont obtenues par incubation des CLC ($1.10^7$ cellules/ml) soit des boîtes de Pétri (Nunc) soit dans des plaques de culture à 6 puits (Nunc) pendant 30 min à 37°C dans un milieu RPMLI 1640 complémenté avec sérum de veau foetal à 10 % (v/v). Les monocytes sont alors récupérés par grattage des plaques après addition d'une solution de PBS froide contenant 1 mM d'EDTA. Les préparations cellulaires comportant plus de 85 % de macrophages viables sont estimées par exclusion au bleu Trypan et coloration avec une estérase non spécifique.

* Culture des macrophages.

Les macrophages sont cultivés à une concentration finale de $2.10^6$ cellules/ml dans des plaques de culture en plastique comportant 12 ou 24 puits (Nunc) et dans un milieu Iscove complémenté avec 100 U/ml de pénicilline, 100 µg/ml de streptomycine et 5% de sérum de veau foetal.

Ce milieu de culture ne contient pas d'endotoxine. Les cellules sont incubées pendant 48 heures à 37°C en atmosphère humide (100 %) contenant 95 % d'air et 5% de $CO_2$, en présence ou en l'absence de 10 µg/ml de β-amyloïde ou de MPTP.

Les surnageants cellulaires sont récupérés pour la mesure du TNF et des nitrites, alors que l'apoptose est évaluée directement sur les cellules.

II. Résultats

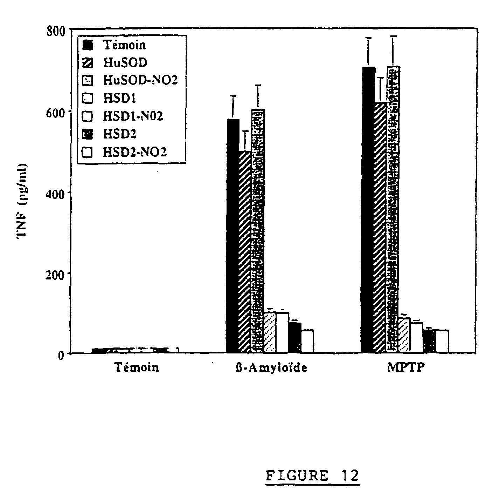

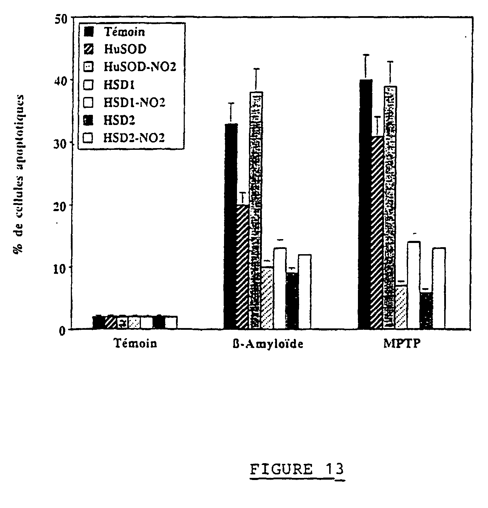

Les figures 12 et 13 illustrent les résultat obtenus.

Ces figures montrent que les protéines β-amyloïde et le MPTP stimulent la production de TNF-α par les macrophages et induisent leur mort par apoptose.

Les HSDs, modifiées ou non, réduisent significativement ces effets, alors que la HuSOD (modifiée ou non) n'a pas ou peu d'effets.

**EXEMPLE 5 : Effets des SODs sur une pathologie dégénérative ; cas de la fibrose radio-induite.**

Le développement de la fibrose apparaît au cours de la plupart des hépatopathies chroniques d'origines variées : infectieuse ou virale (hépatite), parasitaire (bilharziose) ou toxique (alcool, héroïne).

Le tissu fibreux, en se développant, déséquilibre l'unité fonctionnelle de l'organe et agit sur certaines populations cellulaires, principalement les cellules périsinusoïdales, situées dans l'espace de Disse.

La caractérisation des cellules effectrices des fibroses, la régulation des cytokines sur le dépôt matriciel, la standardisation des moyens sériques et tissulaires pour contrôler l'évolution de la maladie chez l'homme permettent d'établir des conduites thérapeutiques afin de limiter, réduire ou arrêter tout processus anormal de fibrogenèse.

Parmi les maladies parasitaires étudiées, la schistosomiase est responsable d'une pathologie hépatique chronique conduisant à la formation d'un dépôt matriciel à l'origine d'une hypertension portale.

Le modèle expérimental de la bilharziose hépatosplénique murine à *Schistosoma mansoni* permet de montrer l'action d'une SOD à activité immuno-redox.

Après infestation des souris, la lésion initiale est une inflammation granulomateuse à médiation cellulaire qui se développe autour des oeufs localisés dans les radicules portaux induisant progressivement une fibrose périovulaire, puis lobulaire, suite à l'activation des cellules hépatiques étoilées (lipocytes), dans l'espace de Disse.

La fibrose évoluant selon un processus dynamique, peut régresser à la suite de certains traitement antifibrosants : cytokines (interféron α, β et/ ou γ), corticostéroïdes ou antioxydants (SOD).

L'activité antifibrosante des SODs a déjà été décrite (Demande Internationale PCT WO 96/16670 et Delanian et al., 1992 et LEFAIX et al., 1996) ; toutefois, il existe toujours le risque d'une action pro-inflammatoire, alors que les SODs selon la présente invention permettent d'éviter un tel risque.

I. Matériel et méthodes

* Animaux : 90 souris IOPS OF1 femelles de 26/28 g (IFFA-CREDO, Les Oncins, L'Arbresle)
* Infestation : chaque souris reçoit, par voie intrapéritonéale, 120 cercaires de la souche Puerto Rico de *Schistosoma mansoni*.

    Protocole 1 :

    1. 5 souris contrôles, non infectées
    2. 5 souris témoins infectées
    3. 25 souris infestées et traitées simultanément pendant 8 semaines, à raison de 3 injections IM/semaine, conformément à la répartition suivante :

    - 5 souris placebos : NaCl à 0,9 %
    - 10 souris : SOD bovine en I.M, 1 mg/kg,
    - 10 souris : SOD bovine en I.M., 2 mg/kg.

    Protocole 2 :

    1. 5 souris contrôles, non infectées
    2. 5 souris témoins infectées

3. 45 souris infestées et traitées simultanément pendant 16 semaines, à raison de 3 injections IM/semaine ou 3 gavages/semaine, conformément à la répartition suivante :

- 5 souris placebo : NaCl à 0,9 %,
- 10 souris : SOD bovine en I.M., 1 mg/kg,
- 10 souris : SOD bovine en I.M., 2 mg/kg,
- 10 souris : SOD de melon en gavage, 2mg/kg,
- 10 souris : SOD de melon en gavage, 4 mg/kg.

* Prélèvements :

. sérum: congélation à -20°C, pour l'étude éventuelle des marqueurs sériques
. foie : un fragment est fixé dans l'AFA (Alcool, Formol, Acide acétique) pour l'étude histologique et morphométrique et un autre fragment est congelé pour les dosages biochimiques.

II. Résultats

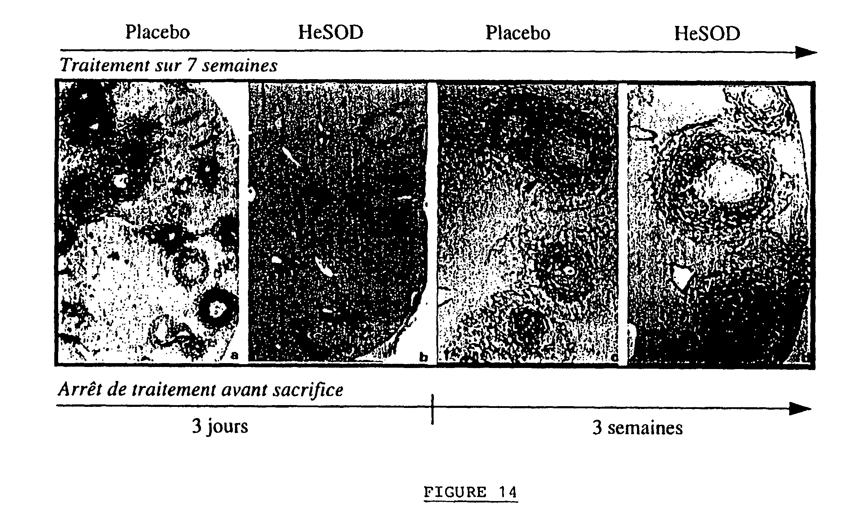

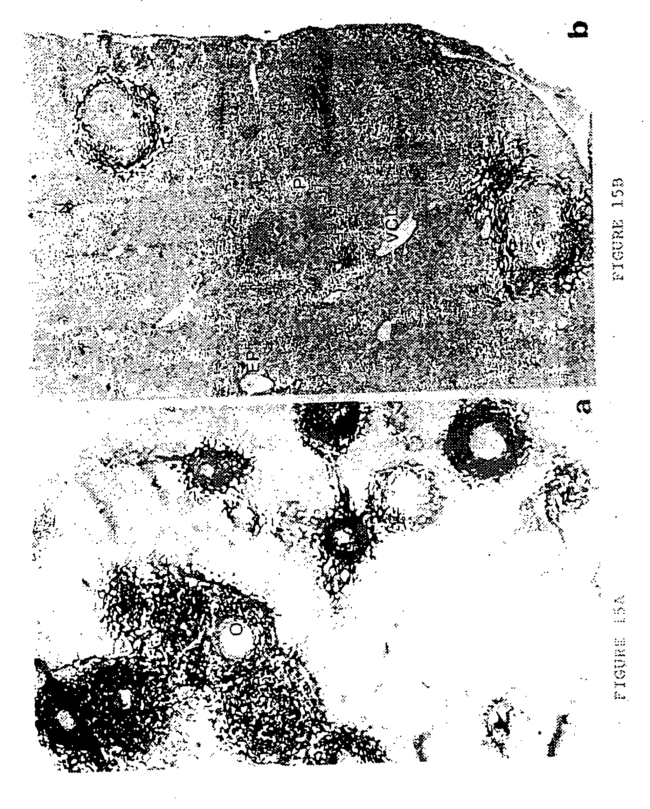

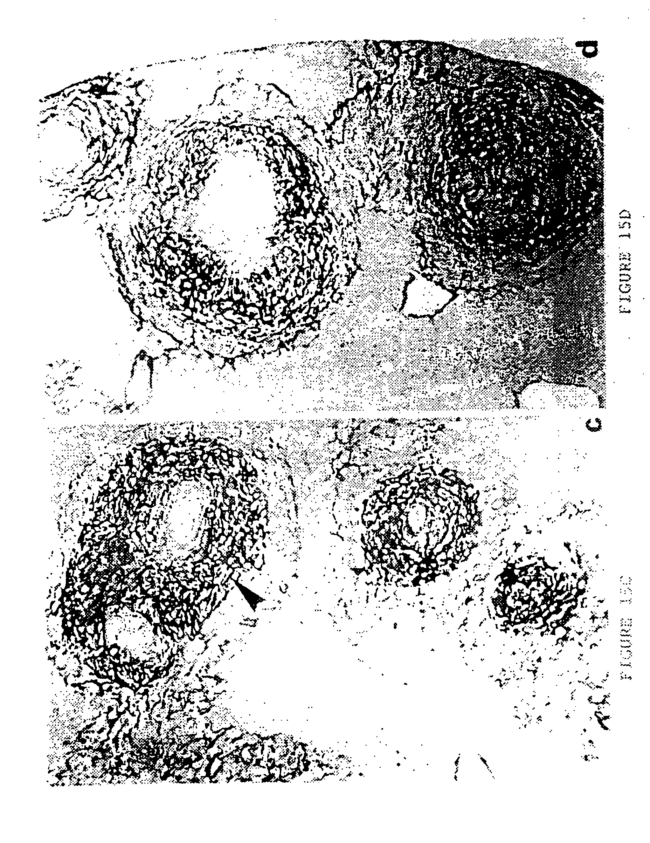

- analyse macroscopique du foie : aspect, répartition des granulomes
- analyse qualitative histologique : étude descriptive des lésions et évaluation semi-quantitative de l'inflammation et de la fibrose, sur coupe histologique de 5 mm, colorées respectivement par l'hémalun-phloxine-safran (figure 14) et le rouge de Picrosirius (figure 15),
- analyse quantitative des dépôts fibreux dans leur ensemble par dosage colorimétrique, basé sur les affinités tinctoriales du rouge Sirius pour le collagène et du fast green pour les protéines ; les résultats sont exprimés en µg de collagène/mg de protéines.

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée, de la présente invention.

**Revendications**

1. Utilisation d'une superoxyde dismutase (SOD) hétérologue végétale ayant une activité de réactivation des défenses cellulaires internes, notamment une stimulation de la production à la fois de SOD endogène, de catalase et de glutathion peroxydase, pour la préparation d'un médicament destiné au traitement des maladies dégénératives.

2. Utilisation selon la revendication 1, **caractérisée en ce que** ladite SOD est nitrée.

3. Utilisation selon la revendication 1 ou la revendication 2, **caractérisée en ce que** ladite SOD est issue du melon.

4. Utilisation selon l'une quelconque des revendications 1 à 3, pour la préparation d'un médicament destiné au traitement des maladies dégénératives sélectionnées dans le groupe constitué par les maladies neurodégénératives, la cirrhose, les infections à lentivirus, les infections parasitaires et les maladies iatrogènes.

5. Procédé de sélection d'une SOD végétale ayant une activité de réactivation des défenses cellulaires internes, notamment une stimulation de la production à la fois de SOD endogène, de catalase et de glutathion peroxydase, **caractérisé en ce qu'**il comprend

(a) la mesure de l'activité dismutasique d'une SOD, nitrée ou non nitrée,

(b) la sélection des SODs sans activité dismutasique ou ayant une activité dismutasique réduite d'au moins un facteur 10, et

(c) la mesure de l'activité de réactivation des défenses cellulaires interne, notamment de stimulation de la production à la fois de SOD endogène, de catalase et de glutathion peroxydase des SODs sélectionnées en (b) sur un système cellulaire humain dans lequel l'expression de SOD endogène est inhibée.

**6.** Procédé selon la revendication 5, **caractérisé en ce que** l'étape (a) de mesure de l'activité dismutasique est réalisée par réduction du ferricytochrome c.

**7.** Procédé selon la revendication 5 ou la revendication 6, **caractérisé en ce que** le système selon l'étape (c) est constitué par des cellules exprimant la protéine tat d'HIV-1.

**8.** Utilisation d'une SOD hétérologue végétale pour la préparation d'un médicament ayant une activité de réactivation des défenses cellulaires internes, notamment de stimulation de la production à la fois de SOD endogène, de catalase et de glutathion peroxydase, destiné au traitement des maladies iatrogènes.

## Patentansprüche

**1.** Verwendung einer heterologen pflanzlichen Superoxiddismutase (SOD), die Aktivität zur Reaktivierung der internen zellulären Abwehr, insbesondere der Stimulierung der gleichzeitigen Produktion von endogener SOD, von Katalase und von Glutathionperoxidase, hat, für die Herstellung eines Arzneimittels, das zur Behandlung degenerativer Krankheiten bestimmt ist.

**2.** Verwendung nach Anspruch 1, **dadurch gekennzeichnet, dass** die SOD nitriert ist.

**3.** Verwendung nach Anspruch 1 oder Anspruch 2, **dadurch gekennzeichnet, dass** die SOD aus der Melone stammt.

**4.** Verwendung nach einem der Ansprüche 1-3 für die Herstellung eines Arzneimittels, das zur Behandlung degenerativer Krankheiten bestimmt ist, welche aus der Gruppe bestehend aus Neurodegenerativen Krankheiten, Zirrhose, Infektionen durch Lentivirus, parasitären Infektionen und iatrogenen Krankheiten ausgewählt sind.

**5.** Verfahren zur Auswahl einer pflanzlichen SOD, die Aktivität zur Reaktivierung der internen zellulären Abwehr, insbesondere der Stimulierung der gleichzeitigen Produktion von endogener SOD, von Katalase und von Glutathionperoxidase, hat, **dadurch gekennzeichnet, dass** es umfasst

(a) Messung der Dismutaseaktivität einer SOD, die nitriert oder nicht nitriert ist,

(b) Auswahl der SODs ohne Dismutaseaktivität oder mit einer Dismutaseaktivität, die mindestens um einen Faktor 10 reduziert ist, und

(c) Messung der Aktivität zur Reaktivierung der internen zellulären Abwehr, insbesondere der Stimulierung der gleichzeitigen Produktion von endogener SOD, von Katalase und von Glutathionperoxidase, der in (b) ausgewählten SODs an einem humanen Zellsystem, in dem die Expression von Endogener SOD inhibiert ist.

**6.** Verfahren nach Anspruch 5, **dadurch gekennzeichnet, dass** die Stufe (a) des Messens der Dismutaseaktivität durch Reduktion des Ferricytochrom c durchgeführt wird.

**7.** Verfahren nach Anspruch 5 oder Anspruch 6, **dadurch gekennzeichnet, dass** das System gemäß Stufe (c) aus Zellen besteht, die das Protein tat von HIV-1 exprimieren.

**8.** Verwendung einer heterologen pflanzlichen SOD für die Herstellung eines Arzneimittels, das Aktivität zur Reaktivierung der internen zellulären Abwehr, insbesondere der Stimulierung der gleichzeitigen Produktion von endogener SOD, von Katalase und von Glutathionperoxidase, hat, das zur Behandlung von iatrogenen Krankheiten bestimmt ist.

## Claims

**1.** Use of a heterologous vegetable superoxide dismutase (SOD) having an activity of reactivation of the internal cell defences, in particular of stimulation of the production at the same time of endogenous SOD, catalase and glutathion peroxidase, for the preparation of a medication intended for the treatment of degenerative illnesses.

**2.** Use according to claim 1, **characterised in that** the said SOD is nitrated.

**3.** Use according to claim 1 or claim 2, **characterised in that** the said SOD comes from melon.

**4.** Use according to any one of claims 1 to 3, for the preparation of a medication intended for the treatment of degenerative illnesses selected from the group consisting of neuro-degenerative illnesses, cirrhosis, lentivirus infections, parasitic infections and iatrogenic illnesses.

**5.** Method of selecting a vegetable SOD having an activity of reactivation of the internal cell defences, in particular of stimulation of he production at the same time of endogenous SOD, catalase and glutathion peroxidase, characterised it comprises

(a) measurement of the dismutasic activity of an SOD, nitrated or non-nitrated,

(b) selection of the SODs without dismutasic activity or having a dismutasic activity reduced by at least a factor of 10, and

(c) measurement of the activity of reactivation of the internal cell defences, in particular of stimulation of the production at the same time of endogenous SOD, catalase and glutathion peroxidase of the selected SODs at (b) on a human cell system in which the expression of endogenous SOD is inhibited.

**6.** Method according to claim 5, **characterised in that** the step (a) of measuring the dismutasic activity is performed by reduction of ferricytochrome c.

**7.** Method according to claim 5 or claim 6, **characterised in that** the system according to step (c) consists of cells expressing the protein tat of HIV-1.

**8.** Use of a heterologous vegetable SOD for the preparation of a medication having an activity of reactivation of the internal cell defences, in particular of stimulation of the production at the same time of endogenous SOD, catalase and glutathion peroxidase, intended for the treatment of iatrogenic illnesses.

SDS-PAGE

FIGURE 1

FIGURE 2

FIGURE 3

FIGURE 4

Superoxide (nmol x $10^6$ cellules)

FIGURE 5

FIGURE 6

FIGURE 7

FIGURE 8

FIGURE 9A

A: Oxyde nitrique

FIGURE 9B

B: Production de cytokines pro-inflammatoires

AZT (µg/ml)

FIGURE 9C

C: Mort cellulaire programmée

% d'Apoptose

AZT (µg/ml)

FIGURE 10A

A: Production d'oxyde nitrique

Nitrites (µM)

FIGURE 10B

B: Production de cytokine pro-inflammatoire

TNF (pg/ml)

FIGURE 10C

C: Mort cellulaire programmee

% de cellules en apoptose

FIGURE 11A

A: Réplication virale

FIGURE 11B

B: Mort cellulaire programmée

FIGURE_12

FIGURE 13

Placebo HeSOD Placebo HeSOD

*Traitement sur 7 semaines*

*Arrêt de traitement avant sacrifice*

3 jours 3 semaines

FIGURE 14

FIGURE 15A

FIGURE 15B

FIGURE 15D

FIGURE 15C

FIGURE 16

FIGURE 17

FIGURE 18